# EUROPEAN PATENT APPLICATION

(11) **EP 0 840 121 A2**
(43) Date of publication of application: **06.05.1998**
(21) Application number: 97308674.7
(22) Date of filing: 30.10.1997
(51) Int. Cl.: G01N 33/32, G01N 17/00

(54) **Device for testing durability of painted surfaces by accelerating wear and method used therefor**

(30) Priority: 05.11.1996 US 29973 P
(71) Applicant: ROHM AND HAAS COMPANY, Philadelphia, Pennsylvania 19106-2399 (US)
(72) Inventor: Romer, Ronald Louis, Jr., Warminster, Pennsylvania 18974 (US)
(74) Representative: Tanner, James Percival

(57) **Abstract**

A device of testing the durability of a surface under accelerated conditions is disclosed. The device provides for testing the surface, such as paint markings from a paint, under simulated environmental conditions which are akin to those experienced by the paint markings applied on the working substrates, such as road surfaces. The device includes one or more wear wheels which roll preferably in a circular motion over a test pattern of the paint markings applied over a test substrate which is similar to a working substrate, such as a road surface. The wear wheel rolls under pressure through a desired number of rotations over the test pattern of paint markings. The pressure is adjusted to simulate the pressure exerted on the actual traffic paint markings on the road surfaces by vehicles driving thereon.

The path of the rolling wear wheels allows the user to simultaneously compare the durability of several experimental paints by exposing each paint marking from the various experimental paints to the same speed and pressure that results in the same degree of wear.

The path of the rolling wear wheels allows the user to simultaneously expose paint markings from several experimental paints to the same degree of wear under the same speed and pressure.

## Description

### Field of Invention

The present invention is directed to testing devices used for measuring the durability of paint markings and, more particularly, to testing devices used for measuring the durability of traffic markings under conditions which substantially simulate actual traffic conditions.

### Background

Paint markings, such as white and yellow traffic markings used for demarcating traffic lanes, is a common sight on almost all roads. These markings ensure safe driving conditions under varying weather conditions. The term "roads" generally means routes, highways, exit and entry ramps, passes, pavements, sidewalks, airport runways, driveways, taxiways or parking lots for vehicles, such as autos, bikes, trucks and airplanes. The roads are usually paved with asphalt or concrete generally made from Portland cement mixed with gravel. The majority of these traffic markings, such as solid, transverse or interrupted stripes, are paint-based, such as those resulting from alkyds, chlorinated rubber-modified alkyds and waterborne acrylic polymer paints. Traditionally, the durability of such paint markings is tested by applying them over road surfaces and then measuring the durability after a test period that typically lasts for a few months to few years. The traffic paints being tested are typically applied over high traffic areas (wheel-track area) of road surfaces from various parts of the country for accelerating the wear action of traffic markings by the vehicular traffic under varied weathered conditions. Such testing methods, including ASTM D713-90 (Standard Practice for Conducting Road Surface Test on Traffic Marking Fluids), however, are time consuming, and recordkeeping of the rate of wear experienced by test traffic markings and the safe inspection thereof is hampered by the presence of vehicular traffic on the road surfaces. Furthermore, due to the long test period, it becomes difficult to quickly select a durable paint from various experimental paint compositions. To offset these problems, traditional laboratory tests, such as applying the coating composition over glass surface and then subjecting them to adhesion test have been tried. Major problems associated with such testing methods is the lack of substantial relationship between the results obtained under the laboratory-based adhesion test on glass surface versus the wear of traffic markings by the vehicular traffic and changing weather conditions typically experienced by the traffic markings on the roads. Thus, it is not possible to predict with a degree of certainty the durability of the traffic paint on the road surface on the basis of the results of the adhesion of traffic paint to glass substrates. See, *Acrylic Lines,* Vol. 7, No. 2, published in June 1996 by Rohm and Haas Company, Philadelphia, Pennsylvania.

Thus, there exists a need for a testing device that can evaluate traffic markings at accelerated rates under conditions that closely simulate vehicular traffic and changing weather conditions to which typical traffic markings are exposed. An article entitled *ROADMAKING PAINT FILM THICKNESS AND RETROREFLECTIVITY RETENTION,* prepared for New Zealand Roadmakers Federation Conference, 26-27 October, 1995, Rotorua, by V. K. Dravtizki (hereafter the Dravtizki article), describes a test device which accelerates the wear of traffic paint by moving in a linear reciprocating motion a pair of weighted test wheels over a test surface coated with traffic markings thereon. The test surface may be subjected to water cycle by means of a timed water irrigation system. However, the testing unit described in the Dravtizki article fails to describe any means for exposing a plurality of traffic markings to the same conditions since as the device travels in a reciprocating motion over the test surface, the middle portion of test track over which the device reciprocates is exposed to maximum velocity as compared to the test tracks located nearer the end of the reciprocating stroke, which are exposed to zero velocity since the test device swings back during its reciprocal motion. Furthermore, the testing unit described in the Dravtizki article fails to simultaneously simulate varied traffic conditions, such as acceleration, deceleration and coasting of vehicles traveling on the road surfaces to which traffic markings are exposed. The present invention solves this problem by providing a testing device that applies the same stresses and shear forces to all of the plurality traffic makings under substantially simulated traffic conditions, such as those working conditions typically experienced by traffic markings on the roads.

### Statement of the Invention

The present invention is directed to a device for testing the durability of a surface of a test bed by wearing said surface at an accelerated rate, said device comprising:
means for wearing said surface comprising at least one wear wheel rotatably mounted on a wheel dolly assembly, said wear wheel being in a rolling contact with said surface; and
means for rotating said wheel dolly assembly to roll said wear wheel through a curved path over said surface.

The present invention is further directed to a method of testing the durability of a surface on a test bed by wearing said surface under accelerated conditions comprising wearing said surface by repeatedly rolling through a curved path at least one wear wheel rotatably mounted on a wheel dolly assembly over said surface.

One of the advantages of the presently claimed testing device includes its ability to apply a desired pressure on the traffic markings applied over a test bed while simultaneously simulating the wear experienced by the traffic markings by the accelerating, coasting and breaking actions produced by vehicles traveling on the road surfaces.

Another advantage of the presently claimed device is its ability to test the durability of paint coatings on surfaces which are substantially identical to the working surfaces, such as road surfaces that may be rough, pitted or dirty.

### Brief Description of Drawings

Fig. 1 illustrates the partially sectioned front elevation of the device of the preferred embodiment of the present invention.

Figs. 2 and 3 illustrate the partially sectioned exploded view of an environmental means and a tray used in the preferred embodiment of the present invention.

Fig. 4 illustrates a three dimensional view of the tray.

Fig. 5 illustrates the plan view of the tray of Fig. 4 containing a test bed having paint markings thereon.

Fig. 6 illustrates a three dimensional view of a shroud of the environmental means of Figs. 1 and 2, which show a dispensing means used in introducing the environmental agents within the space enclosed in the environmental means.

Fig. 7 illustrates the plan view of the shroud shown in Fig. 6.

Fig. 8 is a partial sectional elevational view of a wheel dolly assembly of the device of the preferred embodiment of the present invention shown in Fig. 1.

Fig. 9 is an exploded partial elevational view of a wheel mounting plate of the wheel dolly assembly shown in Fig. 8 to illustrate the engagement of the wheel mounting plate to a collar on a main shaft of the preferred embodiment of the present invention shown in Fig. 1.

Fig. 10 shows a partial elevation of a wear wheel mounted on a wear drive shaft of the wheel dolly assembly shown in Figs. 1 and 8 to illustrate the various portions of the wear wheel that produce a desired pattern of wear on the paint markings shown in Fig. 5.

Fig. 11 illustrates the plan view of the desired pattern of wear on the paint marking shown in Fig. 10.

Fig. 12 illustrates a partial sectional elevation of a wear wheel mounted on a wear drive shaft of the wheel dolly assembly shown in Figs. 1 and 8 for showing the camber added to wear wheel for changing the desired pattern of wear on the paint markings shown in Fig. 5.

Fig. 13 illustrates a partial sectional plan view of a wear wheel mounted on a wear drive shaft of the wheel dolly assembly shown in Figs. 1 and 8 to illustrate the toe-in or toe-out added to wear wheel for changing the desired pattern of wear on the paint markings shown in Fig. 5.

### Detailed Description of the Preferred Embodiment

As used herein:

"Durability" means the resistance of paint to degradation, such as removal, flaking, chipping, cracking of traffic markings, discoloration thereof by black wheel marks or loss of retroreflectivity of the reflective traffic paints.

"Wear" means the degradation of a paint marking applied on surfaces, such as road surfaces, upon exposure to changing weather conditions, such as snow, heat, rain and high winds; vehicular traffic traveling thereon; and abrasive or corrosive action of the environmental agents, such as water, salt, dirt, mud, sand, spilled organic solvents, fuels, lubricants and hydraulic fluids.

The applicant has unexpectedly discovered that the durability of a surface, such as a coating applied over a test bed, can be measured under accelerated conditions, typically in hours or in less than a week, by wearing the surface by repeatedly rolling through a curved path at least one wear wheel over the surface. The device of the present invention is particularly suited to compare and contrast the degradation on test coatings of various paint formulations, such as traffic paints, produced under substantially identical test conditions. These test conditions closely simulate actual working conditions to which markings from such paints are typically exposed.

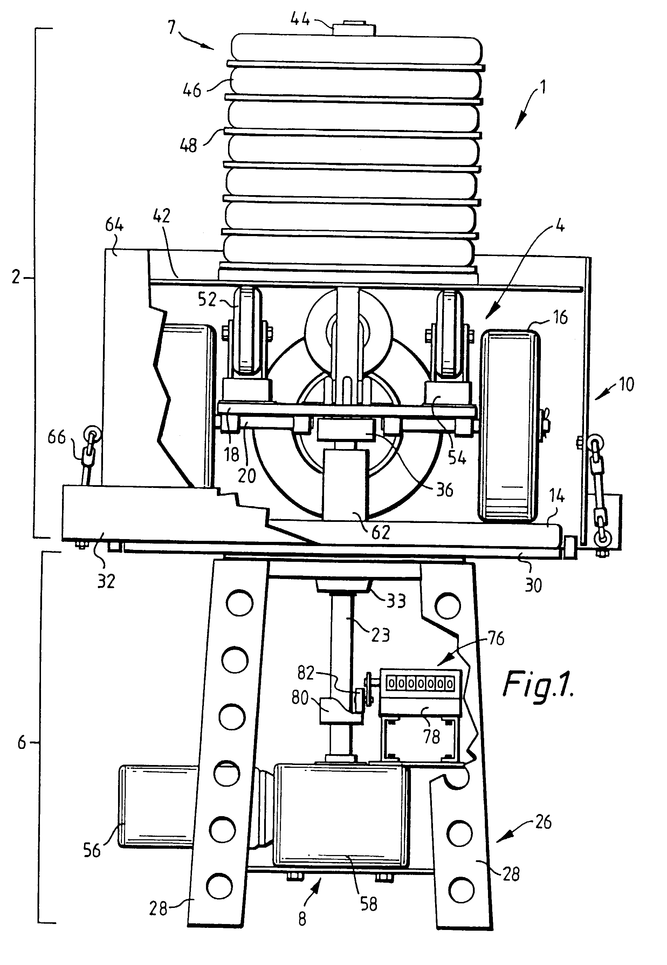

A device identified with a numeral 1 in Fig. 1 illustrates the preferred embodiment of the present invention. As shown in Figs. 1, 5 and 8, device 1 includes means (generally identified with numeral 2) for wearing a surface, such as one or more paint markings 12 applied over a test bed 14, by at least one wear wheel 1G rotatably mounted on a wheel dolly assembly, generally identified with numeral 4 such that wear wheel 16 is in repeated rolling contact with paint markings 12.

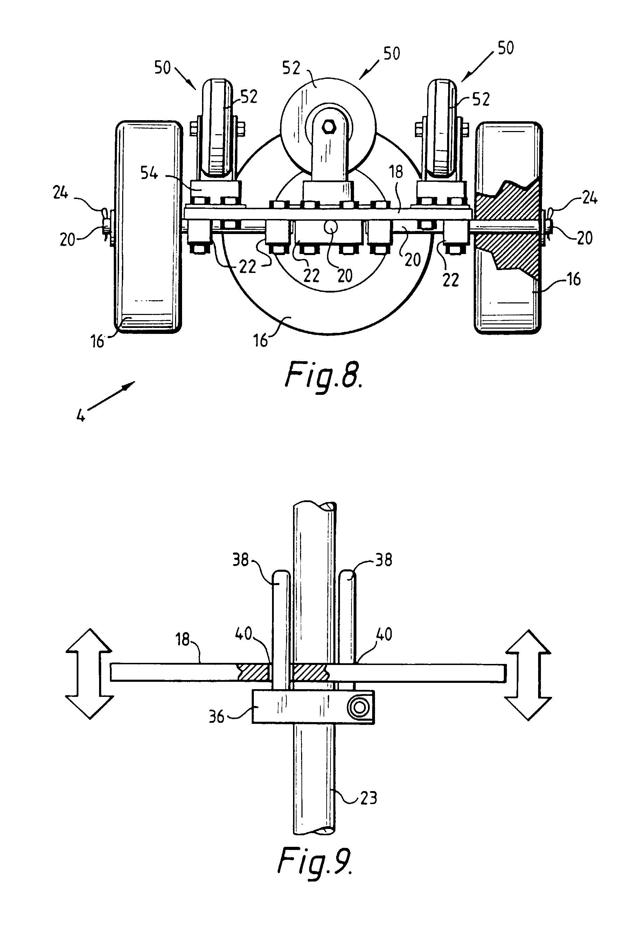

As shown in Figs. 1 and 8, wheel dolly assembly 4 includes a wheel dolly mounting plate 18 having one or more, preferably four, wear wheel drive shafts 20 affixed by wheel dolly securing means, such as nut/bolt assemblies 22. At least one wear wheel 16 is rotatably mounted on the end portion of a wear wheel drive shaft 20 in such a manner that when wheel dolly assembly 4 is placed over test bed 14, wear wheel 16 is in rotational contact with paint markings 12 applied on test bed 14. Thus, when wheel dolly assembly 4 is rotated on a main shaft 23 at a desired revolutions per minute (hereafter rpm), wear wheel 16 rolls in a curved path, preferably in a circular path over paint markings 12 applied on test bed 14. If desired, more wear wheels 16 may be mounted in a staggered fashion on one or more wear wheel drive shaft 20. As shown in Figs. 1 and 8, wheel dolly assembly 4 is preferably provided with four wear wheel drive shafts 20 positioned at 90 degrees apart from each other on wheel dolly mounting plate 18. Each wear wheel drive shaft 20 is preferably provided with one wear wheel 16, such that each wear wheel 16 is 90 degrees apart from the adjacent wear wheel 16. If desired, three wear wheel drive shafts 20 positioned at 120 degrees apart from each other on wheel dolly mounting plate 18 are also suitable, such that each wear wheel 16 is 120 degrees apart from the adjacent wear wheel 16. Wear wheel 16 is secured to wear wheel drive shaft 20 by conventional securing means, such as a split pin 24. Other securing means, such as a collar secured by securing means or such as a set screw set against wear wheel drive shaft 20, is also suitable.

Wear wheel 16 is generally made of a material similar to that used for wheels on vehicles to which traffic markings are typically exposed under normal driving conditions. Thus, wear wheel 16 is generally a pneumatic tire, such as an air inflated tire used on trucks, bicycles, motorcycles, automobiles or airplanes. The surface of wear wheel 16 in rolling contact with paint marking 12 is preferably provided with a wheel tread pattern to closely simulate the wheel wear typically experienced by traffic paints on the road surfaces.

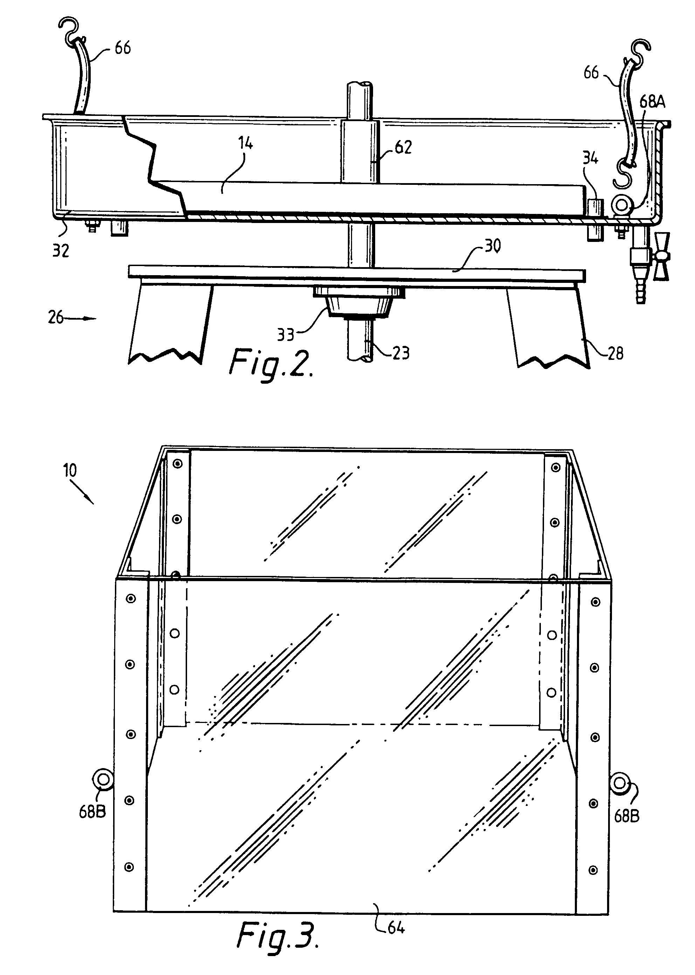

Device 1 includes a frame which is generally indicated by numeral 26. Frame 26 supports substantially all of the components of device 1. Frame 26, which is rigid, is preferably fabricated from flat aluminum stock welded or bolted together to form the supporting structure, having at least three, preferably having four, support legs 28. A rigid platform 30, shown in Figs. 1 and 2, is affixed, preferably by welding, to the upper extremities of frame 26. Platform 30 is preferably a substantially planar metal plate.

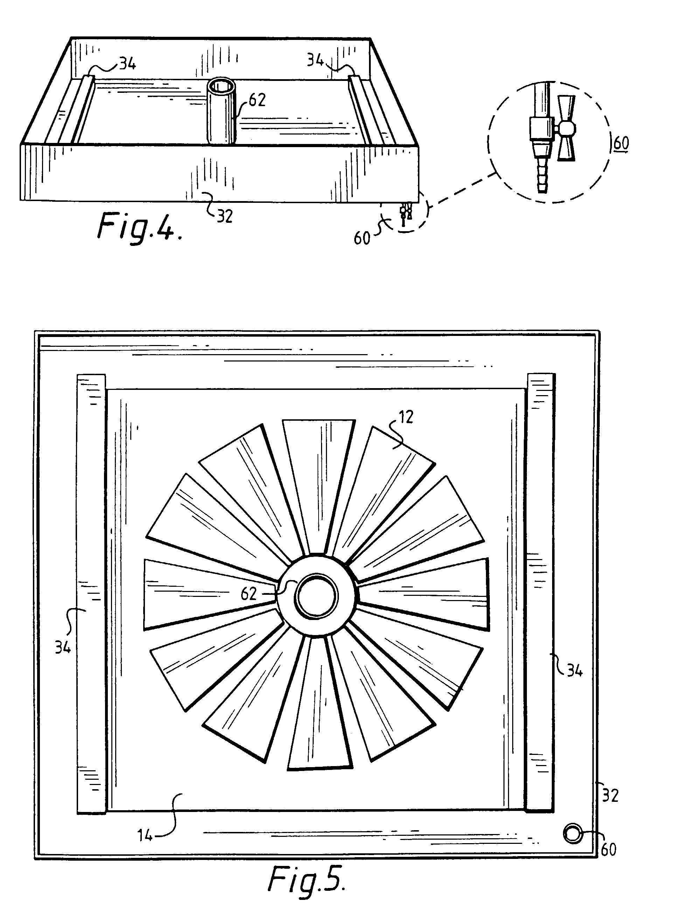

As shown in Figs. 1 and 2, platform 30 supports a tray 32. Tray 32 may be of any desired shape, such as a rectangular or circular shape. A rectangular shape is preferred. Tray 32 is provided with one or more locating means, such as locating bars 34, to align and retain test bed 14 positioned therein. Test bed 14 is provided with one or more paint markings 12, shown in Fig. 5, which are to be tested for wear by device 1. As stated earlier, test bed 14 is substantially identical to substrates, such as wood, metal, concrete, asphalt, resinous or glass substrates, over which the durability of paint markings 12, such as traffic markings, is to be tested. The surface of test bed 14 over which paint markings 12 are applied substantially conforms to the working surface, such as road surface. Platform 30 is provided with a conventional shaft bearing 33 to support main shaft 23 passing therethrough.

As shown in Figs. 1 and 8, main shaft 23 is coupled to wheel dolly assembly 4 to provide power for rotating wheel dolly assembly 4 at a desired rpm. Preferably, wheel dolly assembly 4 is slidably engaged with main shaft 23 to allow wheel dolly assembly 4 to float upwards and downwards as wheel dolly assembly 4 rotates at the desired rpm over paint markings 12. As shown in Figs. 1 and 9, main shaft 23 is preferably provided with a collar 36, which is secured at a desired position on main shaft 23, by suitable securing means, such as a set screw, woodruff key, or shear pin on collar 36. One or more, preferably four, dowel pins 38 are affixed to collar 36. Dowel pins 38 pass through openings 40 provided on a wheel dolly mounting plate 18 of wheel dolly assembly 4. As a result, main shaft 23 can transmit torque to wheel dolly assembly 4, but substantially no thrust load. It is contemplated that other suitable means which allow wheel dolly assembly 4 to float upwards or downwards on main shaft 23 may be employed. For example, main shaft 23 may be provided with splines with correspondingly matched splined openings on wheel dolly mounting plate 18 to accomplish the same floating action of wheel dolly assembly 4 on main shaft 23.

As shown in Fig. 1, wearing means 2 includes force generating means 7 mounted on main shaft 23 and atop wheel dolly assembly 4 for applying a desired pressure on the portions of paint markings 12 in physical contact with wear wheels 16. A weight support plate 42 of force generating means 7 is preferably slidably mounted on main shaft 23. Force generating means 7 is further preferably provided with a centrally disposed sleeve 44 mounted over main shaft 1G and resting atop weight support plate 42. The inner diameter of sleeve 44 is large enough to allow the user to freely install or remove sleeve 44 from main shaft 23. Preferably, sleeve 44 is made of material having low friction to allow force generating means 7 to remain stationary while main shaft 23 rotates within sleeve 44. Sleeve 44 is preferably made of low friction resinous material, such as Teflon® polytetrafluroethylene, supplied by DuPont Company, Wilmington, Delaware.

Force generating means 7 further includes one or more weights 46 of known quantity stacked vertically on top of weight support plate 42 for applying a desired pressure on the portions of paint markings 12 in physical contact with wear wheels 16. Preferably, weight 46 is provided with an opening in the center to allow the user to readily stack one or more weights 46 over sleeve 44. More preferably, one or more vibration absorbent pads 48 are interposed between the adjacent weights 46 to dampen vibrations produced in device 1, when wheel dolly assembly 4 is rotated by the power supplied to main shaft 23. Vibration absorbent pads 48 are preferably provided with lifting slots (not shown) to readily load or unload weights 46 placed thereon. Vibration absorbent pads 48 are made of suitable vibration absorbent material, such as synthetic or natural rubber, or polyolefin, such as propylene. Vibration absorbent pads 48 made from neoprene rubber are preferred.

In order to provide additional support to weights 46 stacked on top of weight support plate 42, resting points are preferably provided, as shown in Figs. 1 and 8, by attaching three or more, preferably four, substantially identical weight distribution means 50 affixed to wheel dolly mounting plate 18. By keeping the height of each weight distribution means 50 above wheel dolly mounting plate 18 substantially the same, weight support plate 42 can be maintained at even level for ensuring substantially balanced and equal weight distribution of the desired force provided by weights 46 stacked atop weight support plate 42. Each weight distribution means 50 preferably includes a caster 52 rotatably mounted on an axle of a caster mounting bracket 54 affixed to wheel dolly mounting plate 18. Thus, when wheel dolly mounting plate 18 is rotated by main shaft 23, caster 52 of weight distribution means 50 can rotate against the surface of weight support plate 42 in contact therewith to keep, if desired, weight support plate 42 and weights 46 mounted thereon, stationary.

It is well within the contemplation of the present invention to substitute weight distribution means 50 by positioning weight support plate 42 on top of a thrust bearing secured to main shaft 23.

It would be apparent to one of ordinary skill in the art that the desired pressure provided by wear wheel 16 on paint markings 12 could also be readily produced by other force generating means, such as by a hydraulic, centrifugal, pneumatic or magnetic device, or by means of a spring operated device, for exerting the desired force on wheel dolly assembly 4.

As shown in Fig. 1, device 1 includes means (generally identified with numeral 6) for rotating wheel dolly assembly 4 to roll wear wheel 16 through a curved path over paint markings 12. Rotating means 6 include driving means 8 fastened to frame 26 to provide power to rotate main shaft 23, positioned in a substantially vertical alignment within frame 26. Driving means 8 includes a power unit 56 which is preferably an electric motor, such as a 3/4 horsepower electric motor Model No. 4K261D supplied by Dayton Electric, Dayton, Ohio. It is understood, however, that depending upon the desired torque and speed, other conventional power units such as an air-driven, gasoline-driven, diesel-driven, or hydraulic motors are also suitable. For high torque/high power requirements, power unit 56 comprising a hydraulic motor is more preferred. Power unit 56 is connected to a main shaft rpm regulating means 58, which is a conventional gear train that can vary the rpm of main shaft 23 to a desired rate. Preferably, main shaft rpm regulating means 58 is a worm gear Model No. M 2614B1-15 supplied by Cleveland Gear, Cleveland, Ohio. It is understood, however, that those of ordinary skill in the art would also consider a chain-, belt-, or air-driven main shaft RPM regulating means 58 as equally suitable. Furthermore, it is understood that a power unit 56 comprising a direct-drive variable-speed power drive would be also suitable.

As shown in Figs. 1, 3, 6 and 7, device 1 is preferably provided with an environmental chamber means 10 positioned atop tray 32. Environmental chamber means 10 is substantially aligned with tray 32 to create an environmental enclosure therewithin. As shown in Figs. 4 and 5, tray 32 is adopted to contain environmental agents which affect the durability of paint markings. Some of such suitable environmental agents include water; muddy water, salt water; solvent; water mixed with oil; mist; hot air; and slurry or suspended abrasive materials, such as sand, mud, clay and dirt. Tray 32 is further provided with draining means, such as a drain valve 60 (details shown in Fig. 4A), to drain, if desired, the liquid environmental agents contained in tray 32. A sleeve 62 is affixed to tray 32 to permit main shaft 23 to pass therethrough.

Environmental chamber means 10 includes a shroud 64, also shown in Figs. 3, 6 and 7, which is preferably open at its upper and lower ends. The lower open end of shroud 64 is preferably contained within tray 32. Preferably, the size of weight support plate 42 is adjusted and shaped to cover the upper open end of shroud 64 to produce the space contained within by environmental chamber means 10. Shroud 64 is preferably fabricated from substantially planar sheets of transparent material to permit observation of the test bed 14 during the test. Shroud 64 is preferably strong enough to withstand repeated use. Some of the transparent materials, suitable for fabricating shroud 64, include polycarbonate sheets or acrylic sheets, such as Plexiglas® supplied by Rohm and Haas Company, Philadelphia, Pennsylvania. As shown in Figs. 2 and 3, shroud G4 is preferably fastened to tray 32 through fastening means that allow easy and quick placement or removal of shroud 64 to and from tray 32. One such suitable fastening means includes one or more, preferably two, stretchable elongated straps 66 having hooks at either end for hooking into eye bolts 68A and 68B, affixed to tray 32 and shroud 64, respectively.

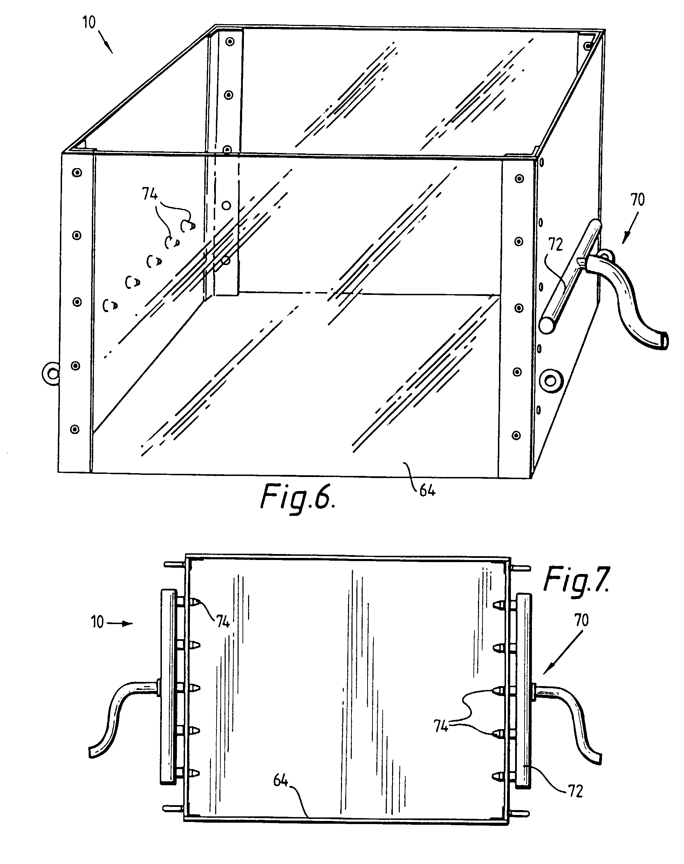

As shown in Figs. 6 and 7, environmental chamber means 10 is further provided with a dispensing means 70 for producing within the space enclosed by environmental chamber means 10, any desired environment including a simulated environment akin to that typically experienced by paint markings. Dispensing means 70 creates the desired environment by introducing the aforedescribed environmental agents in the space enclosed by environmental chamber means 10. One or more manifolds 72 of dispensing means 70 are fastened on the walls of shroud G4 at locations suitable for producing desired environmental conditions within the space enclosed by environmental chamber means 10. A sufficient number of spray nozzles 74 are preferably provided on manifold 72 to dispense, preferably by spraying, one or more of the aforedescribed environmental agents within the space enclosed by environmental chamber means 10. It is well within the contemplation of the present invention to include environment monitoring instruments, such as temperature, air velocity and humidity gages (not shown) to record the environmental conditions within the space enclosed by environmental chamber means 10 and, if desired, utilize the data on such environmental conditions, provided by such monitoring instruments, to control or vary the flow of the environmental agents being dispersed through dispensing means 70 for creating, varying or maintaining the desired environmental conditions within the space enclosed by environmental chamber means 10.

As shown in Fig. 1, device 1 is preferably provided with counter means generally identified with numeral 76 to count and record the number of rotations of main shaft 23 for determining the number of times wear wheels 16 pass over paint markings 12 applied on the surface of test bed 14. Counter means 76, which include a rotation counter 78, preferably Part No. 1895T54 supplied by McMaster-Carr Company, New Brunswick, New Jersey, are mounted on frame 26. A cam 80 positioned on main shaft 23 engages a cam follower 82 connected to the rotation counting arm of counter 78. If desired, the rotations of main shaft 23 may be displayed by conventional display means provided on a display of counter 78.

The applicant also contemplates utilizing a conventional electronic, optical, inductive, magnetic or mechanical counter that sends out a signal that can be stored in the memory of a computer. It is further contemplated that after a fixed number of rotations of main shaft 23, a signal may be generated to shut off the power to power unit 56, thereby permitting hands-off operation of device 1, or after a programmed number of rotations of main shaft 23, a signal may be generated to trigger on or off the introduction of the environmental agents within the space enclosed in environmental means 10.

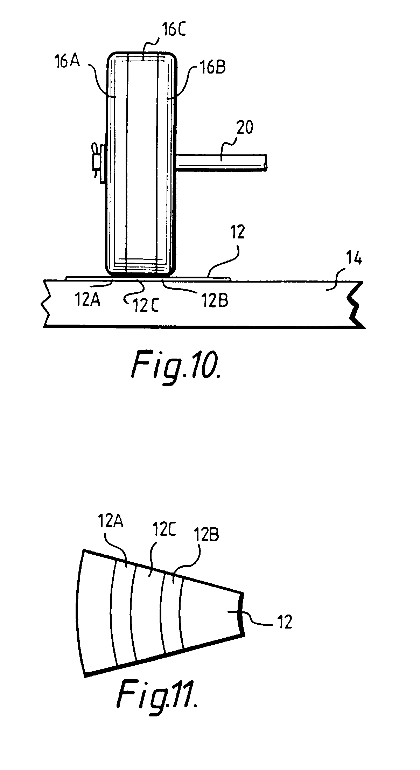

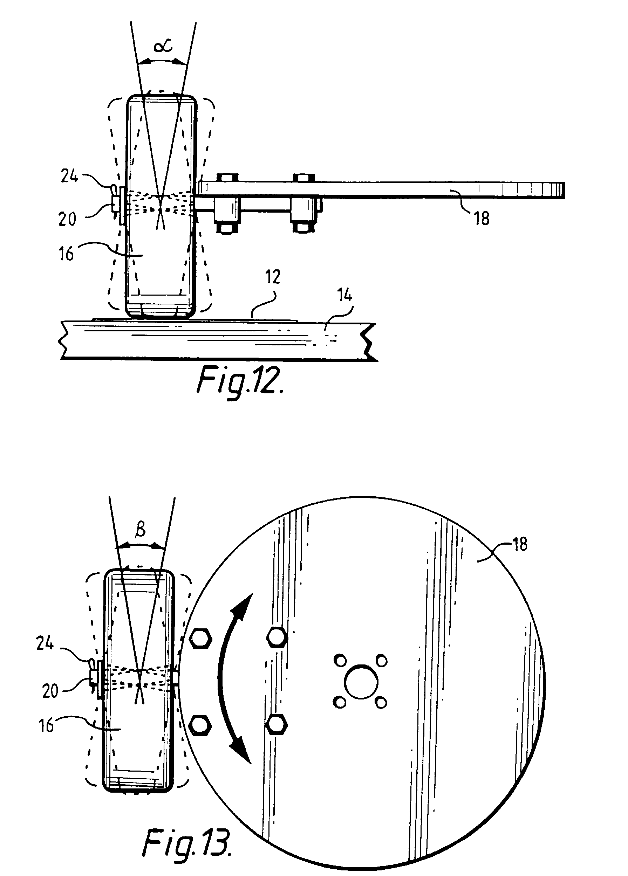

By varying the manner in which wear wheel 1G contacts paint markings 12, a desired pattern of wear of paint markings 12 can be produced. Preferably, wear wheel 16 is positioned in a substantially vertical relationship with paint markings 12, as shown Figs. 10 and 11. As wheel dolly assembly 4 rotates at a desired rpm, wear wheels 16 preferably roll over paint markings 12 in a circular motion. As a result, the outer portion of wear wheel 1GA in contact with the outer portion 12A of paint marking 12, as shown in Fig. 10, moves at a faster speed than the inner portion 16B of wear wheel 16 in contact with the inner portion 12B of paint marking 12. By contrast, there is insignificant relative velocity experienced by the central portion 16C of wear wheel 16 compared to the outer portion 16A or the inner portion 16B of wear wheel 16. As a result, outer portion 12A and inner portion 12B are exposed to shear forces similar to those experienced by traffic markings when motor vehicles decelerate over the traffic markings, such as when the vehicle operator applies brakes to slow down the vehicle or when motor vehicles accelerate over the traffic paint markings. In addition, the central portion 12C of paint marking 12 is subjected to wear that simulates the wear experienced by traffic paint markings when motor vehicles coast over the traffic paint markings on road surfaces at substantially constant speed.

It should be understood that the transition between outer portion 12A to central portion 12C or inner portion 12B of paint marking 12, shown in Figs. 10 and 11, is for illustrative purposes only. The actual transition between these various portions of paint marking 12 is gradual. However, when paint markings 12 from various paint formulations are tested, all such paint markings 12 are exposed to the same shear forces by wear wheels 16. The resulting wear pattern produced on the various positions of paint markings 12 can then be compared and contrasted to determine which paint formulation is more durable. Generally, the area covered by outer portion 12A or by inner portion 12B varies in the range of from 10% to 35 % of the total area of paint marking 12 covered by wear wheel 16.

By providing a bend or otherwise altering the portion of wear wheel drive shaft 20 over which wear wheel 16 rotates, the way in which wear wheel 16 contacts paint markings 12 can be varied, if desired, to change the desired pattern of wear of paint markings 12. Thus, for example, the desired pattern of wear of paint markings 12 may be changed by adding a camber (α), as shown in Fig. 12 or toe-in or toe-out, (β) as shown Fig. 13, to wear wheel 16 in contact with paint markings 12. The camber (α), or toe-in or toe-out (β) changes the desired pattern of wear of paint markings 12 by altering the shear action on the inner portion 12B and outer portions 12A of paint markings 12, as shown in Fig. 10.

In order to operate device 1, the user positions test bed 14 in tray 32 by aligning it by means of locating bars 34. The exposed surface of test bed 14 is coated with paint markings 12 of a desired pattern such as that shown in Fig. 5. Typically, paint markings 12 would be from different paints being tested. If desired, reflective beads, typically used to make traffic markings on the road reflective at night, may also be incorporated in paint markings 12 for testing the loss of retroreflectivity of reflective traffic paint, which occurs when glass beads which make the traffic paint reflective are eroded and removed by the vehicles riding over reflective traffic markings on the road.

Wheel dolly assembly 4 is then slid over main shaft 23 and placed atop the exposed surface of test bed 14 having paint markings 12 applied thereon. The rpm of main shaft 23 may be adjusted to provide the linear speed at which the durability of the traffic paint is to be tested. If desired, the rpm of main shaft 23 maybe adjusted to simulate the number of vehicles passing over traffic paint stripes per minute or per hour. Weights 46 of wear force generating means 7 are adjusted to generate a desired pressure on the portions of the surface of paint markings 12 exposed to wear wheels 16. Typically, by measuring the area of paint marking 12 covered by the surface of wear wheel 16 in contact therewith and by knowing the pressure exerted by loaded or unloaded weights of motor vehicles that travel over the traffic markings on the roads, the amount of weights 46 placed on sleeve 44 can be calibrated to generate the desired pressure on paint markings 12 in device 1. Typically, weights 46 are adjusted to generate in the range of from 6 to 3,500 kilopascals, hereafter kPa,(1 psi to 500 psi), preferably in the range of from 124 psi to 152 kPa pressure over the area on paint markings 12 covered by the portion of wear wheel 16.

Generally, the air pressure in wear wheels 16 is adjusted to a level recommended by the vehicle or wheel manufacturer, which generally varies in the range of from 68.9 kPa to G89 kPa (10 psi to 100 psi), preferably in the range of from 135 kPa to 400 kPa. Alternatively, one may adjust the air pressure in wear wheels 16 to increase or decrease in the portion of paint marking 12 being contacted by wear wheel 16 to a desired area, which is preferably in the range of from 135 kPa to 400 kPa.

The environment within the environmental chamber means 10 is adjusted to simulate desired environmental conditions, such as dry hot, dry cold, damp hot, damp cold, rainy or muddy conditions, by spraying the environmental agents within the space enclosed by environmental chamber means 10.

## Claims

1. A device for testing the durability of a surface of a test bed by wearing said surface at an accelerated rate, said device comprising:
means for wearing said surface comprising at least one wear wheel rotatably mounted on a wheel dolly assembly, said wear wheel being in a rolling contact with said surface; and
means for rotating said wheel dolly assembly to roll said wear wheel through a curved path over said surface.

2. The device of claim 1 wherein said means for wearing said surface further comprise:
means for generating force which is mounted on said wheel dolly assembly such that said wear wheel in use transmits a desired pressure against said surface in contact with said wear wheel.

3. The device of claim 2 wherein said means for generating force comprise one or more weights positioned over said wheel dolly assembly to provide a desired force to said wheel dolly assembly.

4. The device of claim 3 wherein said means for generating force further comprise weight distribution means mounted on a wheel dolly mounting plate of said wheel dolly assembly to substantially balance and distribute said desired force provided by said weights.

5. The device of claim 1 wherein said means for wearing said surface comprise four wear wheels positioned at 90 degrees from each other on a wheel dolly mounting plate of said wheel dolly assembly.

6. The device of claim 1 wherein said wear wheel is a pneumatic tire maintained at a desired pressure.

7. The device of claim 1 wherein said means rotating said wheel dolly assembly further comprise:
driving means slidably engaged to said wheel dolly assembly for rotating said wheel dolly assembly at a desired rate and for floating said wheel dolly assembly upwards or downwards.

8. The device of claim 1 further comprising means for simulating varied weather conditions.

9. A method of testing the durability of a surface on a test bed by wearing said surface under accelerated conditions comprising wearing said surface by repeatedly rolling through a curved path at least one wear wheel rotatably mounted on a wheel dolly assembly over said surface.

10. A method of testing the durability of a paint under accelerated conditions comprising:
applying paint markings of said paint on a test bed;
rolling at a desired pressure and rpm a wear wheel in a circular motion over said paint markings for a desired duration for wearing said paint markings of said paint in a desired pattern under simulated environmental conditions which correspond to environmental conditions to which traffic markings on a road surface are exposed, said desired pattern comprising:
a wear pattern along an outer or inner portion of said paint markings that corresponds to a wear produced on traffic markings on a road by a vehicle traveling under deceleration or acceleration thereon,
a wear pattern along a central portion of said paint markings that corresponds to a wear produced on traffic markings on a road by a vehicle coasting thereon; and
measuring the area of said paint markings remaining on said test bed.
